# EUROPEAN PATENT APPLICATION

(11) **EP 2 604 173 A1**
(43) Date of publication of application: **19.06.2013**
(21) Application number: 12776623.6
(22) Date of filing: 18.04.2012
(51) Int. Cl.: A61B 1/00, A61B 1/06, A61B 5/1459, G01N 21/27

(54) **OPTICAL MEASURING DEVICE**

(30) Priority: 26.04.2011 US 201161479108 P
(71) Applicant: Olympus Medical Systems Corp., Tokyo 151-0072 (JP)
(72) Inventor: KAMIMURA, Kenji, Tokyo 151-0072 (JP)
(74) Representative: Schicker, Silvia
(86) International application number: PCT/JP2012/060468
(87) International publication number: WO 2012/147585

(57) **Abstract**

An optical measurement 1 apparatus according to the present invention includes: an irradiation fiber 5; a plurality of light receiving fibers 7 to 9; a light source unit 22 that generates and outputs light to irradiate a biological tissue 6 and supplies the light to the irradiation fiber 5; a measurement unit 23 that has a plurality of measurement units and performs spectrometry on each returned light from the biological tissue, the returned light output by each of the light receiving fibers 7 to 9; and a control unit 26 that causes a predetermined storage unit 27 to store a measurement result by the measurement unit 23, and the measurement unit 23 sequentially performs pre-characteristic-value-obtainment measurement of the returned light under a predetermined condition after power is supplied and, in the pre-characteristic-value-obtainment measurement, starts spectrometry for characteristic value obtainment when an increase in a received light intensity is detected, the increase being an amount set based on an amount of supplied light from the light source unit 22 and a light reflection or scattering state of the biological tissue 6.

## Description

### Field

The present invention relates to an optical measurement apparatus that performs spectrometry on returned light reflected or scattered by a biological tissue and obtains a characteristic value of the biological tissue.

### Background

In recent years, an optical measurement apparatus has been proposed, which uses a low-coherence enhanced backscattering (LEBS) technique for detecting characteristics of a biological tissue by irradiating the biological tissue, which is a scatterer, with low-coherence light having a short spatial coherence length from a distal end of a probe and measuring a distribution of its scattered light intensity (for example, see Patent Literature 1 to 4). Such an optical measurement apparatus performs optical measurement on an object to be measured such as a biological tissue or the like in combination with an endoscope that observes internal organs such as digestive organs.

The optical measurement apparatus using this LEBS technique obtains the intensity distribution of the scattered light of the biological tissue by obtaining scattered light beams of a plurality of desired angles with a plurality of light receiving fibers and thereafter performing spectrometry with a plurality of measurement devices respectively, and obtains characteristic values related to characteristics of the biological tissue based on results of this measurement.

### Citation List

### Patent Literature

Patent Literature 1: International Patent Publication Pamphlet No. WO 2007/133684
Patent Literature 2: U.S. Patent Application Laid-open No. 2008/0037024
Patent Literature 3: U.S. Patent No. 7652881
Patent Literature 4: U.S. Patent Application Laid-open No. 2009/0003759

### Summary

### Technical Problem

In the above-mentioned conventional optical measurement apparatus, each measurement device is caused to start measurement in predetermined timing by giving a predetermined synchronization trigger signal to each measurement device. Therefore, it is necessary to provide, outside the measurement devices, a trigger generating circuit for generating the synchronization trigger signal and, in each measurement device, a trigger circuit that receives the synchronization trigger signal and drives the measurement device and, thus there is a problem that an apparatus configuration of the optical measurement apparatus becomes complicated.

The present invention has been made in view of the above, and aims to provide an optical measurement apparatus capable of causing a plurality of measurement devices to start measurement in appropriate timing, as well as achieving simplification of the apparatus configuration.

### Solution to Problem

To solve the above problem and achieve the object, an optical measurement apparatus according to the present invention performs spectrometry on returned light reflected or scattered by a biological tissue and obtains a characteristic value of the biological tissue and the optical measurement apparatus includes an irradiation fiber that conducts light supplied from a proximal end thereof and irradiates the light from a distal end thereof; a plurality of light receiving fibers that each conduct light entering from a distal end thereof and outputs the light from a proximal end thereof; a light source unit that generates and outputs light to irradiate the biological tissue and supplies the light to the irradiation fiber; a plurality of measurement devices that are provided as many as the plurality of light receiving fibers and respectively perform spectrometry on returned light from the biological tissue, the returned light respectively output by the plurality of light receiving fibers; and a control unit that causes a predetermined storage unit to store each measurement result from the plurality of measurement devices. Each measurement device sequentially performs pre-characteristic-value-obtainment measurement of the returned light under a predetermined condition after power is supplied and, in the pre-characteristic-value-obtainment measurement, starts spectrometry for characteristic value obtainment when an increase in a received light intensity is detected, the increase being an amount set based on an amount of supplied light from the light source unit and a light reflection or scattering state of the biological tissue. The control unit causes the storage unit to store a measurement result after each measurement device starts the measurement for characteristic value obtainment, the measurement result being data necessary to obtain the characteristic value of the biological tissue.

Moreover, in the optical measurement apparatus according to the present invention, the light source unit supplies the light such that the received light intensities of the returned light in the plurality of measurement devices are distinguishable from an intensity of ambient light.

Moreover, in the optical measurement apparatus according to the present invention, the light source unit temporarily increases the amount of supplied light at start of irradiation, and in the pre-characteristic-value-obtainment measurement, each measurement device starts the spectrometry for the characteristic value obtainment when a temporary increase in the received light intensity is detected, the temporary increase corresponding to the temporary increase in the amount of supplied light at the start of irradiation in the light source unit.

Moreover, in the optical measurement apparatus according to the present invention, the light source unit includes a light source that emits light; and a shutter that is provided on an optical path of light emitted from the light source and is freely openable and shuttable under control of the control unit.

Moreover, in the optical measurement apparatus according to the present invention, each measurement device starts the spectrometry for the characteristic value obtainment when an increase in received light intensity for light of a predetermined wavelength is detected in the pre-characteristic-value-obtainment measurement.

Moreover, in the optical measurement apparatus according to the present invention, the light source unit is able to change a wavelength of light output, which is irradiation light, and irradiates light of the predetermined wavelength during a predetermined time period from the start of irradiation.

Moreover, in the optical measurement apparatus according to the present invention, the light source unit includes a white light source that emits white light, a filter that transmits light of the predetermined wavelength included in the white light, and a transport unit that transports the filter, and the control unit causes the transport unit to transport the filter onto an optical path before the pre-characteristic-value-obtainment measurement.

### Advantageous Effects of Invention

With an optical measurement apparatus according to the present invention, each measurement device performs, under a predetermined condition after power is supplied, pre-characteristic-value-obtainment measurement of returned light that is reflected or scattered by a biological tissue and, in the pre-characteristic-value-obtainment measurement, automatically starts spectrometry for characteristic value obtainment when an increase in a received light intensity is detected, the increase being an amount set based on an amount of supplied light from a light source unit and a light reflection or scattering state of an object to be measured; and therefore, it is possible to cause a plurality of measurement devices to start measurement in appropriate timing without providing a trigger generating circuit and a trigger circuit.

### Brief Description of Drawings

FIG. 1 is a schematic diagram illustrating a schematic configuration of an optical measurement apparatus according to an embodiment of the present invention.
FIG. 2 is a diagram illustrating installation of a probe illustrated in FIG. 1 to an endoscope.
FIG. 3 is a block diagram illustrating a configuration of a measurement unit illustrated in FIG. 1.
FIG. 4 is a diagram illustrating a processing sequence of a measurement process of a first measurement unit illustrated in FIGS. 1 and 3.
FIG. 5 has a diagram illustrating time dependence of output light intensity from a light source unit illustrated in FIG. 1 and a diagram illustrating time dependence of received light intensity measured by a first measurement unit illustrated in FIG. 3.
FIG. 6 is a diagram illustrating time dependence of received light intensity measured by the first measurement unit illustrated in FIG. 3 when a biological tissue and a distal end of the probe are not appropriately in contact with each other.
FIG. 7 has a diagram illustrating another example of time dependence of output light intensity from the light source unit illustrated in FIG. 1 and a diagram illustrating another example of time dependence of received light intensity measured by the first measurement unit illustrated in FIG. 3.
FIG. 8 is a schematic diagram illustrating another schematic configuration of an optical measurement apparatus according to an embodiment of the present invention.
FIG. 9 is a schematic diagram illustrating another schematic configuration of an optical measurement apparatus according to an embodiment of the present invention.

### Description of Embodiments

Hereinafter, preferred embodiments of an optical measurement apparatus according to the present invention will be described in detail with reference to the drawings. The present invention is not limited by these embodiments. In the description of drawings, like reference numerals denote like elements. Further, it is to be noted that the drawings are schematic, and relations between thicknesses and widths of each element, and ratios among elements are different from those of the actual. Among the drawings also, a same portion having relations or ratios of dimensions different from one another is included.

### (First Embodiment)

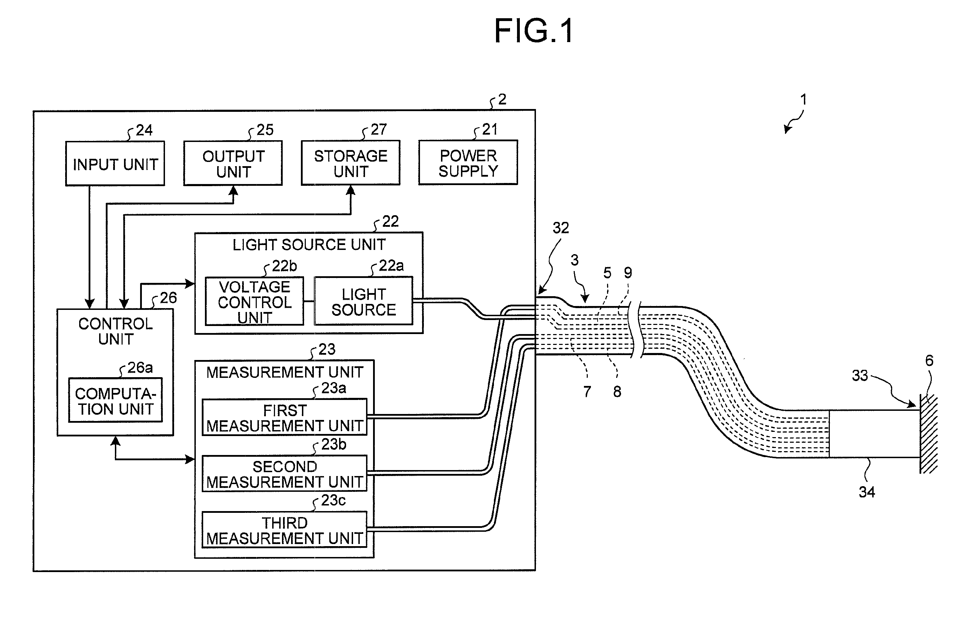

FIG. 1 is a schematic diagram illustrating a schematic configuration of an optical measurement apparatus according to an embodiment of the present invention. As illustrated in FIG. 1, an optical measurement apparatus 1 according to the embodiment includes a main unit 2 that performs optical measurement on a biological tissue 6, which is an object to be measured, and detects characteristics of the biological tissue 6, and a probe 3 for measurement, which is inserted into a subject. The probe 3 has flexibility, a proximal end 32 thereof is detachably connected to the main unit 2, light supplied from the proximal end 32 is emitted from a distal end 33 thereof to the biological tissue 6 by the main unit 2 connected, and reflected light and scattered light entering from the distal end 33, which are returned light from the biological tissue 6, are output from the proximal end 32 to the main unit 2.

The main unit 2 includes a power supply 21, a light source unit 22, a measurement unit 23, an input unit 24, an output unit 25, a control unit 26, and a storage unit 27.

The power supply 21 supplies power to each element of the main unit 2.

The light source unit 22 generates and outputs light to be irradiated onto the biological tissue 6. The light source unit 22 is implemented using a light source 22a, which is a low-coherence light source such as a white light emitting diode (LED) that emits white light, a xenon lamp, or a halogen lamp, one or more lenses (not illustrated), and a voltage control unit 22b that controls a voltage amount to be supplied to the light source 22a. The light source unit 22 supplies the low-coherence light to be irradiated onto an object to an irradiation fiber 5 of the probe 3 described below.

The measurement unit 23 performs spectrometry on the returned light from the biological tissue 6, which is light output from the probe 3. The measurement unit 23 is implemented using a plurality of spectrometers. The measurement unit 23 measures a spectral component, intensity, and the like of the returned light output from the probe 3 and performs measurement per wavelength. The measurement unit 23 outputs a result of the measurement to the control unit 26. The measurement unit 23 is provided with the same number of spectrometers as that of a plurality of light receiving fibers of the probe 3 described below. In an example illustrated in FIG. 1, a first measurement unit 23a, a second measurement unit 23b, and a third measurement unit 23c, corresponding to a plurality of light receiving fibers 7 to 9 of the probe 3 and each having a spectrometer, are included.

The input unit 24 is implemented using a push-type switch and the like, receives instruction information for instructing activation of the main unit 2 and various other types of instruction information, and inputs them to the control unit 26.

The output unit 25 outputs information related to various processes in the optical measurement apparatus 1. The output unit 25 is implemented using a display, a speaker, a motor, or the like, and outputs information related to various processes in the optical measurement apparatus 1 by outputting image information, audio information, or vibration.

The control unit 26 controls processing operations of each element of the main unit 2. The control unit 26 includes a CPU and a semiconductor memory such as a RAM. The control unit 26 controls operations of the main unit 2 by transferring or the like instruction information and data to respective elements of the main unit 2. The control unit 26 causes the storage unit 27 described below to store each measurement result from the measurement unit 23 having a plurality of measurement devices. The control unit 26 includes a computation unit 26a.

The computation unit 26a performs various types of computation processes based on the measurement result by the measurement unit 23 and computes a characteristic value related to characteristics of the biological tissue 6. The type of the characteristic value that is computed by the computation unit 26a and is a target to be obtained is set according to instruction information input from the input unit 24 through manipulation by an operator.

The storage unit 27 stores an optical measurement program that causes the main unit 2 to perform an optical measurement process and various types of information related to the optical measurement process. The storage unit 27 stores each measurement result by the measurement unit 23. In addition, the storage unit 27 stores the characteristic value computed by the computation unit 26a.

The probe 3 has the proximal end 32 detachably connected to a predetermined connector of the main unit 2 and the distal end 33 from which light supplied from the light source unit 22 is emitted and into which scattered light from an object to be measured enters. The probe 3 is implemented using one or more optical fibers. When the LEBS technique is used, a plurality of light receiving fibers are provided to receive at least two scattered light beams having different scattering angles. Specifically, the probe 3 includes the irradiation fiber 5 that conducts light from the light source unit 22 supplied from the proximal end 32 to irradiate from the distal end 33 onto the biological tissue 6 and three light receiving fibers 7 to 9 that each conduct scattered light and reflected light from the biological tissue 6, which enter from the distal end 33, to output from the proximal end 32. At distal ends of the irradiation fiber 5 and the light receiving fibers 7 to 9, a rod 34 having transparency is provided. The rod 34 has a cylindrical shape such that distances between a surface of the biological tissue 6 and the distal ends of the irradiation fiber 5 and light receiving fibers 7 to 9 become constant. Although in the example illustrated in FIG. 1, the probe 3 has three light receiving fibers 7 to 9, two or more light receiving fibers are sufficient since ability to receive at least two scattered light beams having different scattering angles is sufficient. Further, an example in which the three light receiving fibers 7 to 9 are of the same standard will be explained.

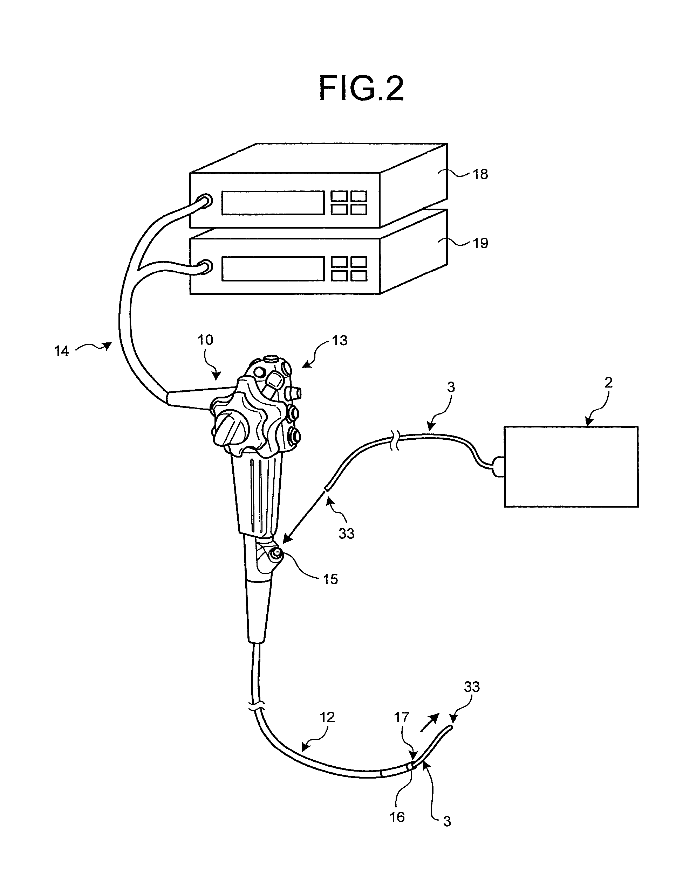

The optical measurement apparatus 1 is used in combination with an endoscope system that observes internal organs such as digestive organs. FIG. 2 is a diagram illustrating installation of the probe 3 to an endoscope. In FIG. 2, a flexible universal cord 14 extending from a lateral portion of a manipulation unit 13 of an endoscope 10 is connected to a light source device 18 and a signal processor 19 that processes a subject image captured at a distal end portion 16 of the endoscope 10. The probe 3 is inserted from a probe channel insertion hole 15 in the vicinity of the manipulation unit 13 of an out-of-body portion of the endoscope 10 inserted into a subject. The probe 3 passes inside an insertion portion 12 and the distal end 33 protrudes from an aperture 17 of the distal end portion 16 connected to a probe channel. As a result, the probe 3 is inserted into the subject, and the optical measurement apparatus 1 starts optical measurement on the biological tissue 6.

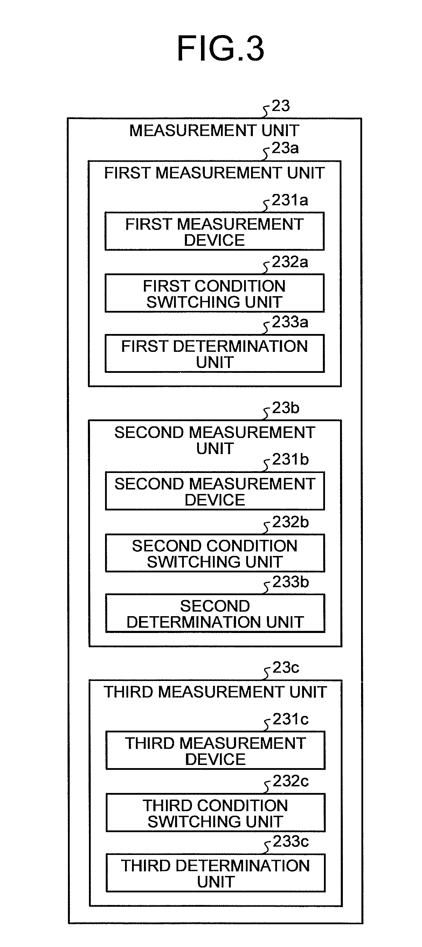

Next, a configuration of the measurement unit 23 illustrated in FIG. 1 will be described in detail. FIG. 3 is a block diagram illustrating a configuration of the measurement unit 23 illustrated in FIG. 1. As illustrated in FIG. 3, the first measurement unit 23a includes a first measurement device 231a, a first condition switching unit 232a, and a first determination unit 233a.

The first measurement device 231a is a spectrometer that performs spectrometry on returned light from a biological tissue output by the light receiving fiber 7.

The first condition switching unit 232a switches a measurement condition of the first measurement device 231a. The first condition switching unit 232a switches the measurement condition of the first measurement device 231a to a measurement condition of spectrometry for characteristic value obtainment performed to obtain a characteristic value of a biological tissue or to a measurement condition of pre-characteristic-value-obtainment measurement performed before the spectrometry for the characteristic value obtainment. The measurement condition of the spectrometry for the characteristic value obtainment is to perform spectrometry on wavelengths of an entire rage of a measurement range of returned light set for the characteristic value obtainment. The measurement condition of the pre-characteristic-value-obtainment measurement is to perform intensity measurement on only light having a predetermined wavelength included in white light. For example, in the pre-characteristic-value-obtainment measurement, the intensity measurement is performed on a wavelength less than 500 nm. Since a white light source is used in the optical measurement apparatus 1, in the pre-characteristic-value-obtainment measurement, the intensity measurement is performed on the wavelength less than 500 nm included in the white light, that is, any wavelength belonging to a blue light wavelength range of 400 to 500 nm.

The first condition switching unit 232a, after power is supplied to the measurement unit 23, sets the measurement condition of the first measurement device 231a to the measurement condition of the pre-characteristic-value-obtainment measurement such that the pre-characteristic-value-obtainment measurement is first performed, and causes the first measurement device 231a to sequentially perform the pre-characteristic-value-obtainment measurement. Measurement results of the pre-characteristic-value-obtainment measurement are sequentially output to the first determination unit 233a. Further, the first condition switching unit 232a switches the measurement condition of the first measurement device 231a from the measurement condition of the pre-characteristic-value-obtainment measurement to the measurement condition of the spectrometry for the characteristic value obtainment when switching of the measurement condition is instructed from the first determination unit 233a.

The first determination unit 233a causes the first condition switching unit 232a to switch the measurement condition of the first measurement device 231a from the measurement condition of the pre-characteristic-value-obtainment measurement to the measurement condition of the characteristic value obtainment measurement when an increase in received light intensity is detected, in the measurement results of the received light intensity of the pre-characteristic-value-obtainment measurement, the increase being an amount set based on an amount of supplied light from the light source unit 22 and a light reflection state or a light scattering state of the biological tissue. Specifically, the first determination unit 233a causes the first condition switching unit 232a to switch the measurement condition of the first measurement device 231a from the measurement condition of the pre-characteristic-value-obtainment measurement to the measurement condition of the characteristic value obtainment measurement when the received light intensity measured in the pre-characteristic-value-obtainment measurement becomes equal to or greater than a predetermined threshold value. This predetermined threshold value is obtained by adding, to received light intensity of ambient light, an increment of received light intensity of the amount set based on the amount of supplied light from the light source unit 22 and the light reflection or scattering state of the biological tissue.

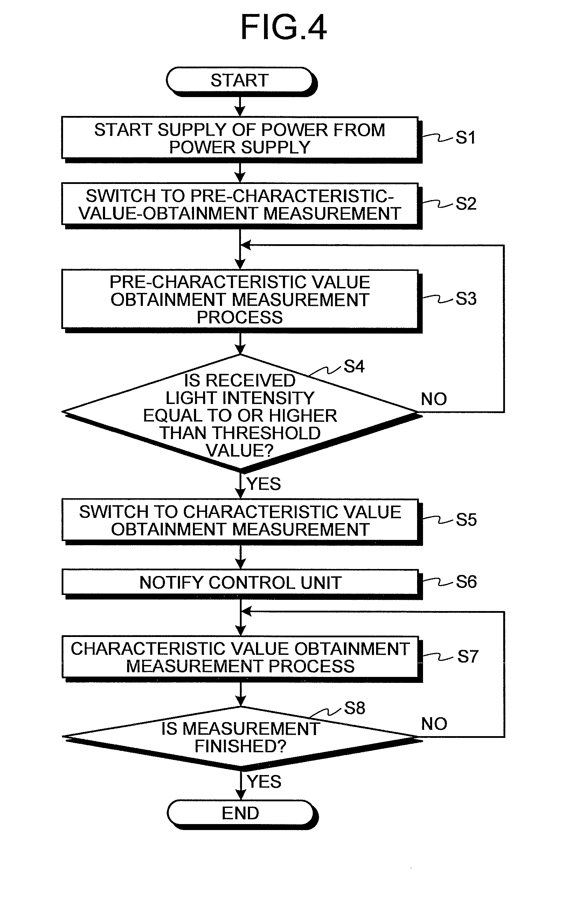

Next, a measurement process of the first measurement unit 23a will be described. FIG. 4 is a diagram illustrating a processing sequence of the measurement process of the first measurement unit 23a illustrated in FIGS. 1 and 3.

As illustrated in FIG. 4, as supply of power from the power supply 21 to the first measurement unit 23a is started (step S1), the first condition switching unit 232a first switches the measurement condition of the first measurement device 231a to the measurement condition of the pre-characteristic-value-obtainment measurement (step S2), and the first measurement device 231a executes the pre-characteristic-value-obtainment measurement process of performing intensity measurement on only light having a predetermined wavelength (step S3). This received light intensity measured in the pre-characteristic-value-obtainment measurement process is output to the first determination unit 233a.

The first determination unit 233a determines whether the received light intensity measured in the pre-characteristic-value-obtainment measurement process has become equal to or greater than a predetermined threshold value (step S4). If the first determination unit 233a determines that the received light intensity measured in the pre-characteristic-value-obtainment measurement has not become equal to or greater than the predetermined threshold value (NO in step S4), the process returns to step S3, and the pre-characteristic-value-obtainment measurement process is continued.

If the first determination unit 233a determines that the received light intensity measured in the pre-characteristic-value-obtainment measurement process has become equal to or greater than the predetermined threshold value (YES in step S4), the first determination unit 233a causes the first condition switching unit 232a to switch the measurement condition of the first measurement device 231a from the measurement condition of the pre-characteristic-value-obtainment measurement to the measurement condition of the characteristic value obtainment measurement (step S5) and notifies the control unit 26 that the measurement condition of the first measurement device 231a has been switched to the measurement condition of the characteristic value obtainment measurement (step S6). Subsequently, the first measurement device 231a executes a characteristic value obtainment measurement process of performing spectrometry on wavelengths of the entire range of the measurement range of returned light set for the characteristic value obtainment (step S7). The measurement results of the characteristic value obtainment measurement process are output to the control unit 26. The control unit 26 causes the storage unit 27 to store the measurement results after the first measurement device 231a starts the characteristic value obtainment measurement process, which are data necessary to obtain a characteristic value of the biological tissue. Further, the computation unit 26a computes the characteristic value of the biological tissue based on the measurement results of the characteristic value obtainment measurement process and causes the storage unit 27 to store the characteristic value computed.

Thereafter, the first determination unit 233a determines whether finishing of the measurement has been instructed based on the instruction information input from the control unit 26 (step S8). If the first determination unit 233a determines that finishing of the measurement has not been instructed (NO in step S8), the process returns to step S7, and the characteristic value obtainment measurement process is continued. If the first determination unit 233a determines that finishing of the measurement has been instructed (YES in step S8), the measurement process in the first measurement unit 23a is finished.

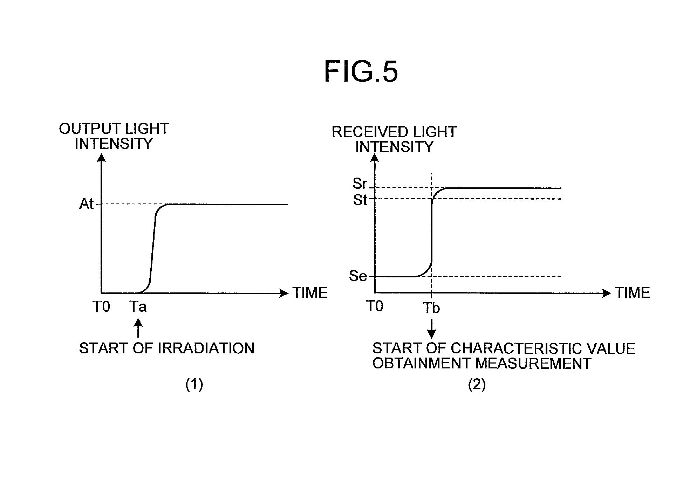

Next, the predetermined threshold value determined by the first determination unit 233a in step S4 of FIG. 4 will be described. FIG. 5(1) is a diagram illustrating time dependence of output light intensity from the light source unit 22. FIG. 5(2) is a diagram illustrating time dependence of received light intensity measured in the first measurement device 231a.

The light source unit 22 is set such that irradiation of light starts at time Ta after a predetermined time elapses from time T0 at which power supply from the power supply 21 starts. From the light source unit 22, for example as illustrated in FIG. 5(1), light of intensity At set as output light intensity in the characteristic value obtainment measurement is output at the time Ta. The light having the intensity At output from the light source unit 22 is irradiated onto a biological tissue via the irradiation fiber 5. Thereafter, light reflected and scattered by the biological tissue enters each of the light receiving fibers 7 to 9, and intensity of a light beam of the light entering the light receiving fiber 7, the light beam having a predetermined wavelength, is measured by the first measurement device 231a.

The light source unit 22 outputs light with intensity such that the received light intensity for the returned light from the biological tissue measured by the first measurement device 231a is distinguishable from intensity Se of ambient light such as irradiation light from the endoscope 10 and external light. Specifically, the light source unit 22 outputs light with the same intensity as the intensity At of the light output in the characteristic value obtainment measurement. When the biological tissue and the distal end 33 of the probe 3 are appropriately in contact with each other, received light intensity of a light beam of returned light from the biological tissue corresponding to light output from the light source unit 22 with the intensity At, the light beam having a predetermined wavelength, increases from the intensity Se of the ambient light to intensity Sr, which has increased to an amount corresponding to an amount of supplied light from the light source unit 22 and the light reflection or scattering state of the biological tissue and has a sufficient difference from the intensity Se of the ambient light as illustrated in FIG. 5(1).

Therefore, if the received light intensity measured by the first measurement device 231a becomes equal to or greater than a threshold value St set based on this intensity Sr and a measurement processing speed, measurement accuracy, and the like of the first measurement device 231a, supply of light corresponded to the characteristic value obtainment measurement from the light source unit 22 is considered to have started. Therefore, the first determination unit 233a causes the first condition switching unit 232a to switch the measurement condition of the first measurement device 231a from the measurement condition of the pre-characteristic-value-obtainment measurement to the measurement condition of the characteristic obtainment measurement at time Tb at which the received light intensity equal to or greater than the threshold value St is detected in the measurement results of the received light intensity of the pre-characteristic-value-obtainment measurement. As a result, the first measurement device 231a starts the characteristic value obtainment measurement from this time Tb.

Further, a second measurement device 231b is a spectrometer that performs spectrometry on returned light from the biological tissue output by the light receiving fiber 8. The second measurement unit 23b includes a second measurement device 231b having a function same as that of the first measurement device 231a, a second condition switching unit 232b, and a second determination unit 233b as illustrated in FIG. 3, and executes a processing sequence similar to each of the processing sequence illustrated in FIG. 4. The second condition switching unit 232b has a function similar to that of the first condition switching unit 232a, and when switching of a measurement condition is instructed from the second determination unit 233b, the second condition switching unit 232b switches the measurement condition of the second measurement device 231b from a measurement condition of the pre-characteristic-value-obtainment measurement of measuring intensity of light having a predetermined wavelength to a measurement condition of spectrometry for the characteristic value obtainment to measure wavelengths of the entire range of a measurement range of the returned light. Similarly to the first determination unit 233a, the second determination unit 233b causes the second condition switching unit 232b to switch the measurement condition of the second measurement device 231b from the measurement condition of the pre-characteristic-value-obtainment measurement to the measurement condition of the characteristic value obtainment measurement when the received light intensity exceeding a threshold value St is detected in the measurement results of the received light intensity of the pre-characteristic-value-obtainment measurement. The light receiving fiber 8 is of the same standard as that of the light receiving fiber 7. Therefore, in the second measurement unit 23b, the second measurement device 231b starts the characteristic value obtainment measurement in approximately the same timing as the timing in which the first measurement device 231a starts the characteristic value obtainment measurement.

Further, a third measurement device 231c is a spectrometer that performs spectrometry on returned light from the biological tissue output by the light receiving fiber 9. The third measurement unit 23c includes a third measurement device 231c having a function same as that of the first measurement device 231a, a third condition switching unit 232c, and a third determination unit 233c, and executes a processing sequence similar to each of the processing sequence illustrated in FIG. 4. The third condition switching unit 232c has a function similar to that of the first condition switching unit 232a. When switching of the measurement condition is instructed from the third determination unit 233c, the third condition switching unit 232c switches the measurement condition of the third measurement device 231c from the measurement condition of the pre-characteristic-value-obtainment measurement of measuring intensity of light having a predetermined wavelength to the measurement condition of spectrometry for the characteristic value obtainment to measure wavelengths of the entire range of a measurement range of the returned light. Similarly to the first determination unit 233a, the third determination unit 233c causes the third condition switching unit 232c to switch the measurement condition of the third measurement device 231c from the measurement condition of the pre-characteristic-value-obtainment measurement to the measurement condition of the characteristic value obtainment measurement when the received light intensity exceeding a threshold value St is detected in the measurement results of the received light intensity in the pre-characteristic-value-obtainment measurement. The light receiving fiber 9 is of the same standard as that of the light receiving fiber 7. Therefore, in the third measurement unit 23c, the third measurement device 231c starts the characteristic value obtainment measurement in approximately the same timing as the timing in which the first measurement device 231a starts the characteristic value obtainment measurement.

In this manner, the first measurement unit 23a, the second measurement unit 23b, and the third measurement unit 23c in the measurement unit 23 of the optical measurement apparatus 1 according to the embodiment perform the pre-characteristic-value-obtainment measurement of the reflected light under a predetermined condition after power is supplied, and in this pre-characteristic-value-obtainment measurement, automatically start the spectrometry for the characteristic value obtainment when the increase in the received light intensity is detected, the increase being the amount set based on the amount of supplied light from the light source unit 22 and the light reflection or scattering state of the object to be measured. Accordingly, with the optical measurement apparatus 1, it is possible to cause the plurality of first, second, and third measurement units 23a, 23b, and 23c to start their measurements approximately simultaneously in appropriate timing without providing a trigger generating circuit outside the measurement devices and a trigger circuit inside the measurement devices, and thus it is possible to simplify the apparatus configuration.

Further, in the optical measurement apparatus 1, the first, second, and third measurement units 23a, 23b, and 23c of the measurement unit 23 determine switching of the measurement condition based on whether the received light intensity in the pre-characteristic-value-obtainment measurement has become equal to or greater than a predetermined threshold value. This threshold value is set by adding, to the received light intensity of the ambient light, an increase in the received light intensity, the increase being the amount set based on the amount of supplied light from the light source unit 22 and the light reflection or scattering state of the biological tissue. Therefore, the first, second, and third measurement units 23a, 23b, and 23c of the measurement unit 23 determine the switching of the measurement condition based on whether there is the increase in the received light intensity in the pre-characteristic-value-obtainment measurement. As a result, in the optical measurement apparatus 1, even in a state in which ambient light such as endoscope illumination light or external light is naturally present, it is possible to appropriately switch the measurement condition.

Further, according to the optical measurement apparatus 1, in the pre-characteristic-value-obtainment measurement, the intensity is measured only for a predetermined wavelength belonging to the blue light wavelength range, instead of wavelengths of the entire range within a measurable range, and thus a simple process until the start of the characteristic value obtainment measurement is sufficient. In addition, if the biological tissue is in an appropriate state, approximately constant reflection and scattering characteristics are exhibited regardless of types of internal organs, and thus it is not necessary to separately set, for each internal organ, the threshold value to be compared with the measurement result of the characteristic value obtainment measurement.

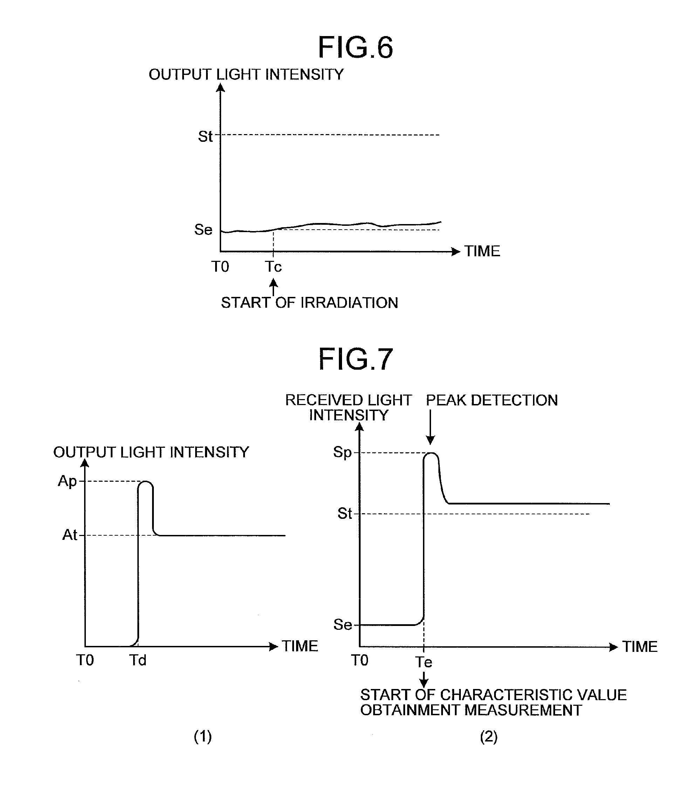

When the biological tissue and the distal end 33 of the probe 3 are not appropriately in contact with each other, the received light intensity of the returned light almost does not increase from the intensity Se of the ambient light although irradiation of light onto the biological tissue has started from time Tc as illustrated in FIG. 6. In other words, if the biological tissue and the distal end 33 of the probe 3 are appropriately in contact with each other, the received light intensity of the returned light is able to increase to a threshold value or greater. Therefore, in the optical measurement apparatus 1, it is possible to detect that the biological tissue and the distal end 33 of the probe 3 are appropriately in contact with each other by just detecting that there has been an increase in the pre-characteristic-value-obtainment measurement to a value equal to or greater than a predetermined threshold value, without verifying a state of contact between the biological tissue and the distal end 33 of the probe 3 using another device such as an endoscope. Furthermore, in the optical measurement apparatus 1, because only the measurement result measured in a state in which the biological tissue and the distal end 33 of the probe 3 are appropriately in contact with each other is stored, and the characteristic value is computed based on only the measurement results measured in a state in which the biological tissue and the distal end 33 of the probe 3 are appropriately in contact with each other, it is possible to sufficiently ensure reliability of the measurement results and the characteristic value of the biological tissue.

Biological tissues naturally have high reflectance of red light having a wavelength belonging to a red wavelength range. When there is irradiation light from an endoscope or external light, intensity of red light in reflected light caused by ambient light increases, and as a result of setting a measurement range of received light intensity of each measurement device of the measurement unit 23 wide, an increase in received light intensity of the red light of returned light from a biological tissue becomes small with respect to the measurement range of the received light intensity, and the increase of the received light intensity of the red light of the returned light may become difficult to distinguish. In the optical measurement apparatus 1, the measurement unit 23 determines the switching of the measurement condition based on whether there is the increase in the received light intensity of the blue light belonging to the blue wavelength range having low reflectance from the biological tissue. Since the intensity of the blue light due to the ambient light which naturally exists is low, it is not necessary to aimlessly widen the measurement range of the received light intensity of each measurement device of the optical measurement apparatus 1, and the optical measurement apparatus 1 is able to sufficiently distinguish the increase in the received light intensity of the blue light included in the returned light from the biological tissue.

In addition, in the optical measurement apparatus 1, for the pre-characteristic-value-obtainment measurement sequentially performed, as long as the increase in the received light intensity of the returned light from the biological tissue is able to be verified, the increase being equal to or greater than a predetermined amount, the measurement unit 23 may perform a differential computation process for the received light intensity measured by each measurement device and determine whether there is the increase in the received light intensity, the increase being equal to or greater than a predetermined amount, based on whether there is a steep rise in values computed.

In addition, the light source unit 22 of FIG. 1 may change the light emission amount of the light source 22a by the voltage control unit 22b changing a supplied voltage value to the light source 22a. That is, the light source unit 22 may change the amount of supplied light. As illustrated in FIG. 7(1), the light source unit 22 temporarily increases the amount of supplied light up to intensity Ap (> At) at irradiation start time Td, and the first, second, and third measurement units 23a, 23b, and 23c start the spectrometry for the characteristic value obtainment based on time Te at which the temporary increase in the received light intensity corresponding to the temporary increase in the amount of supplied light at the irradiation start time of the light source unit 22, that is, a peak of the received light intensity, is detected in the pre-characteristic-value-obtainment measurement. In this case, if the measurement unit 23 performs a differential computation process for the received light intensity, it becomes easier to detect the steep rise in the values computed.

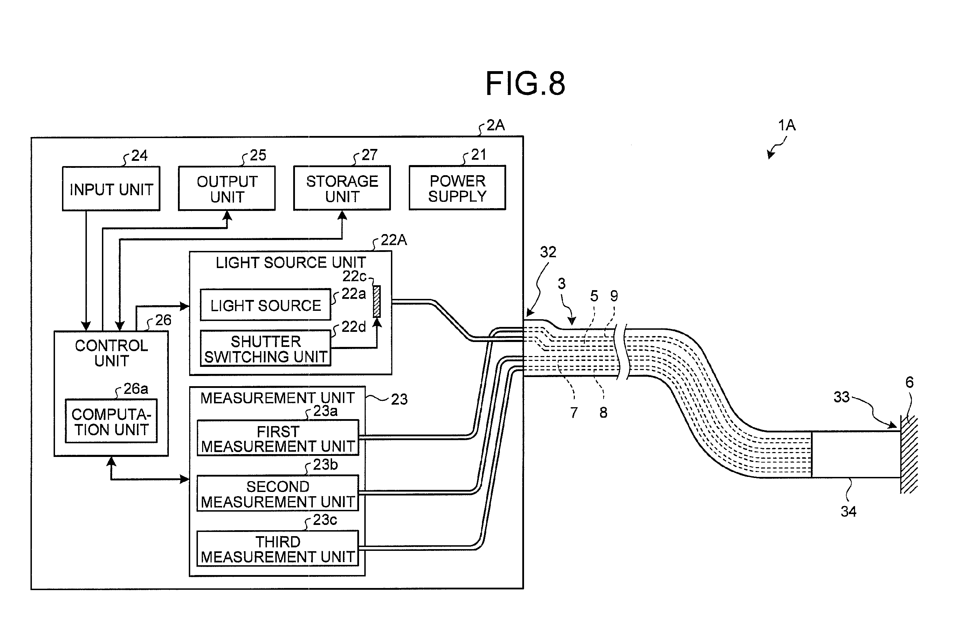

Instead of the light source unit 22 provided with the voltage control unit 22b illustrated in FIG. 1, like a main unit 2A of an optical measurement apparatus 1A illustrated in FIG. 8, a light source unit 22A may be provided with a light source 22a, a shutter 22c provided on an optical path of light emitted from the light source 22a, and a shutter switching unit 22d that switches between opening and shutting of the shutter 22c. The shutter 22c is freely openable and shuttable by switching between opening and shutting by the shutter switching unit 22d under control of the control unit 26. In this case, as a voltage allowing the light source 22a to output light of an intensity At is supplied to the light source unit 22A, the control unit 26 causes the shutter switching unit 22d to shut the shutter 22c until the pre-characteristic-value-obtainment measurement starts and causes the shutter switching unit 22d to open the shutter 22c when the pre-characteristic-value-obtainment measurement is started.

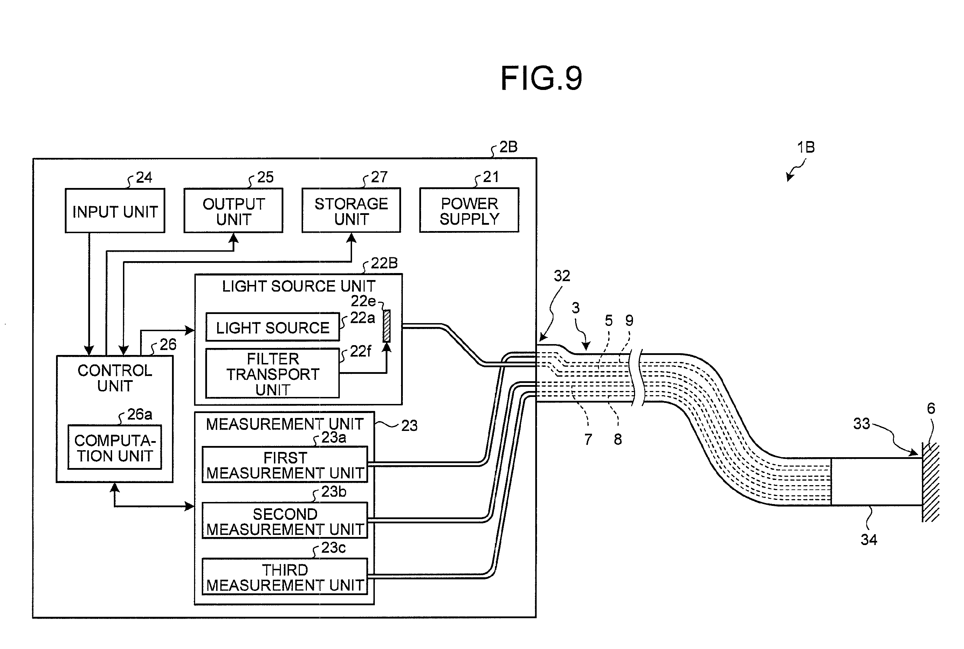

In one embodiment, measurement efficiency may be improved by causing a light source unit to output only a predetermined wavelength belonging to the blue light wavelength range, the predetermined wavelength being a determination target for a received light intensity change by the measurement unit 23 during a predetermined time period in which the pre-characteristic-value-obtainment measurement is performed after irradiation starts. Specifically, like a main unit 2B of an optical measurement apparatus 1B illustrated in FIG. 9, a light source unit 22B including a light source 22a emitting white light, a movable filter 22e that transmits only a predetermined wavelength included in white light and belonging to the blue light wavelength range, and a filter transport unit 22f that transports the filter 22e are provided. The control unit 26 causes the filter transport unit 22f to transport the filter 22e onto the optical path of white light from the light source 22a before the pre-characteristic-value-obtainment measurement is performed. Further, the control unit 26 causes the filter transport unit 22f to retreat the filter 22e from the optical path of white light from the light source 22a before the characteristic value obtainment measurement is performed.

Further, a blue light source that emits light of a blue wavelength range may be provided in addition to the light source 22a which is a white light source, white light may be overlapped with blue light in the pre-characteristic-value-obtainment measurement, and the received light intensity of blue light at the measurement unit 23 side may be increased.

Although description has been made for an example in which the measurement unit 23 measures the received light intensity of a predetermined wavelength belonging to the blue light wavelength range in the pre-characteristic-value-obtainment measurement, limitation is of course not made thereto, and a wavelength to be a measured for received light intensity may be appropriately selected from wavelengths included in white light, depending on a measurement sensitivity of the measurement unit 23 and the like.

Further, in the pre-characteristic-value-obtainment measurement, the measurement unit 23 may measure the received light intensity for the wavelengths of the entire range of the measurable range and determine the switching of the measurement condition by comparing integrated values of the received light intensity of this range with a predetermined threshold value set beforehand. If the wavelengths of the entire range corresponding to a visible light band are measurable, the measurement unit 23 may measure the received light intensity for the wavelengths of the entire range of the visible light band, perform an integration process, and compare the integrated values with a threshold value to determine the start of the characteristic value obtainment measurement.

### Reference Signs List

1, 1A, 1B optical measurement apparatus
2, 2A, 2B main unit
3 probe
5 irradiation fiber
6 biological tissue
7 to 9 light receiving fiber
10 endoscope
12 insertion portion
13 manipulation unit
14 universal cord
15 probe channel insertion hole
16 distal end portion
17 aperture
18 light source device
19 signal processor
21 power supply
22, 21A, 22B light source unit
22a light source
22b voltage control unit
22c shutter
22d shutter switching unit
22e movable filter
22f filter transport unit
23 measurement unit
23a first measurement unit
23b second measurement unit
23c third measurement unit
24 input unit
25 output unit
26 control unit
26a computation unit
27 storage unit
32 proximal end
33 distal end
34 rod
231a first measurement device
231b second measurement device
231c third measurement device
232a first condition switching unit
232b second condition switching unit
232c third condition switching unit
233a first determination unit
233b second determination unit
233c third determination unit

## Claims

1. An optical measurement apparatus that performs spectrometry on returned light reflected or scattered by a biological tissue and obtains a characteristic value of the biological tissue, the optical measurement apparatus comprising:
an irradiation fiber that conducts light supplied from a proximal end thereof and irradiates the light from a distal end thereof;
a plurality of light receiving fibers that each conduct light entering from a distal end thereof and outputs the light from a proximal end thereof;
a light source unit that generates and outputs light to irradiate the biological tissue and supplies the light to the irradiation fiber;
a plurality of measurement devices that are provided as many as the plurality of light receiving fibers and respectively perform spectrometry on returned light from the biological tissue, the returned light respectively output by the plurality of light receiving fibers; and
a control unit that causes a predetermined storage unit to store each measurement result from the plurality of measurement devices, wherein
each measurement device sequentially performs pre-characteristic-value-obtainment measurement of the returned light under a predetermined condition after power is supplied and, in the pre-characteristic-value-obtainment measurement, starts spectrometry for characteristic value obtainment when an increase in a received light intensity is detected, the increase being an amount set based on an amount of supplied light from the light source unit and a light reflection or scattering state of the biological tissue, and
the control unit causes the storage unit to store a measurement result after each measurement device starts the measurement for characteristic value obtainment, the measurement result being data necessary to obtain the characteristic value of the biological tissue.

2. The optical measurement apparatus according to claim 1, wherein the light source unit supplies the light such that received light intensities of the returned light in the plurality of measurement devices are distinguishable from an intensity of ambient light.

3. The optical measurement apparatus according to claim 1, wherein
the light source unit temporarily increases the amount of supplied light at start of irradiation, and
in the pre-characteristic-value-obtainment measurement, each measurement device starts the spectrometry for the characteristic value obtainment when a temporary increase in the received light intensity is detected, the temporary increase corresponding to the temporary increase in the amount of supplied light at the start of irradiation in the light source unit.

4. The optical measurement apparatus according to claim 1, wherein the light source unit includes:
a light source that emits light; and
a shutter that is provided on an optical path of light emitted from the light source and is freely openable and shuttable under control of the control unit.

5. The optical measurement apparatus according to claim 1, wherein each measurement device starts the spectrometry for the characteristic value obtainment when an increase in received light intensity for light of a predetermined wavelength is detected in the pre-characteristic-value-obtainment measurement.

6. The optical measurement apparatus according to claim 5, wherein the light source unit is able to change a wavelength of light output, which is irradiation light, and irradiates light of the predetermined wavelength during a predetermined time period from the start of irradiation.

7. The optical measurement apparatus according to claim 6, wherein
the light source unit includes a white light source that emits white light, a filter that transmits light of the predetermined wavelength included in the white light, and a transport unit that transports the filter, and
the control unit causes the transport unit to transport the filter onto an optical path before the pre-characteristic-value-obtainment measurement.
